# FASCICULE DE BREVET EUROPEEN

(11) **EP 3 439 540 B1**
(45) Date de publication et mention de la délivrance du brevet: **07.06.2023**
(21) Numéro de dépôt: 17714498.7
(22) Date de dépôt: 05.04.2017
(51) Int. Cl.: A61B 5/00, A61B 5/0215, A61B 5/02, A61F 2/90

(54) **DISPOSITIF MÉDICAL MUNI DE CAPTEURS**
MIT SENSOREN AUSGESTATTETE MEDIZINISCHE VORRICHTUNG
MEDICAL DEVICE PROVIDED WITH SENSORS

(30) Priorité: 06.04.2016 FR 1653032
(43) Date de publication de la demande: 13.02.2019
(73) Titulaire: Sensome, 91300 Massy (FR)
(72) Inventeur: BOZSAK, Franz, 78000 VERSAILLES (FR); CARREEL, Bruno, 75014 PARIS (FR); MESSINA, Pierluca, 75012 PARIS (FR); COTTANCE, Myline, 94310 ORLY (FR)
(74) Mandataire: Icosa
(86) Numéro de dépôt international: PCT/EP2017/058169
(87) Numéro de publication internationale: WO 2017/174688

(56) Documents cités:
- DE-A1- 10 103 503
- US-A1- 2005 065 592
- US-A1- 2006 047 205
- US-A1- 2011 054 583
- US-A1- 2012 190 989
- US-A1- 2014 343 382
- Robert Daniel: "Les registres à décalage", , 30 septembre 2005 (2005-09-30), XP055381302, Extrait de l'Internet: URL:http://electronique-et-informatique.fr /Digit/Digit_8T.html#SORTIES [extrait le 2017-06-13]
- Anonymous: "Ring oscillator - Wikipedia", , 1 août 2015 (2015-08-01), XP055381299, Extrait de l'Internet: URL:https://en.wikipedia.org/w/index.php?t itle=Ring_oscillator&oldid=674008095 [extrait le 2017-06-13]

## Description

La présente invention concerne un dispositif médical muni de capteurs. L'invention vise également un système médical comprenant un tel dispositif médical et un procédé d'interrogation d'un tel dispositif médical, notamment dans un système médical.

L'invention vise notamment un dispositif médical implantable tel un stent (parfois également appelé « endoprothèse artérielle », « tuteur vasculaire » voire même simplement « ressort ») muni de capteurs.

Un stent est un dispositif de forme tubulaire réalisé par un maillage déformable, notamment en métal ou en un matériau polymère biodégradable. Le stent est introduit dans le corps d'un patient dans un état replié, mailles fermées, puis est étendu à l'intérieur du corps du patient, par exemple par angioplastie qui provoque le déploiement des mailles. Le stent, déployé, permet de maintenir ouverte une cavité dans le corps du patient. Il est connu que la mise en place d'un stent peut notamment provoquer une inflammation des tissus, une hyperplasie et/ou une coagulation de sang.

Par conséquent, un stent peut être muni de capteurs, qui permettent de surveiller l'état des tissus autour du stent pour, éventuellement, adapter le traitement du patient en conséquence. Des capteurs peuvent également être prévus pour s'assurer que le stent remplit sa fonction de maintien d'une cavité ouverte.

Il est connu d'interroger un stent, c'est-à-dire de recueillir une information provenant de ce stent, à l'aide d'un dispositif d'interrogation sans contact, conservé à l'extérieur du patient. Généralement, un tel dispositif d'interrogation sans contact est conformé pour mesurer un champ électromagnétique émis par le stent implanté dans le patient.

Le brevet EP-B-2 271 933 décrit ainsi un procédé pour caractériser des cellules au voisinage d'un dispositif médical implanté dans un patient, notamment un stent, par des mesures d'impédance à différentes fréquences.

La demande WO-A-2009/1 361 677 décrit un dispositif médical implantable tel qu'un stent, présentant une surface conductrice d'électricité et un capteur d'impédance pour mesurer l'impédance de la surface conductrice du dispositif médical implantable, à différentes fréquences, en utilisant la surface conductrice comme électrode. Les mesures effectuées sont utilisées pour déterminer le degré de resténose des tissus au niveau du dispositif implantable, c'est-à-dire l'épaisseur de tissu ayant poussé au niveau de la surface conductrice du dispositif médical implantable.

Ces documents enseignent des méthodes donnant une information globale sur le dispositif implantable, sans permettre d'obtenir indépendamment les mesures réalisées par chaque capteur dont est muni le dispositif médical implantable.

Par ailleurs, il est connu de US-B-8 478 378, un stent muni de capteurs répartis sur sa surface interne, orientée vers le passage à travers le stent, ou surface « luminale ». Les capteurs sont configurés pour envoyer un signal de sortie caractéristique propre en réponse à une excitation. Le signal caractéristique propre peut notamment être une longueur d'onde propre à chacun des capteurs. US-B-8 478 378 indique qu'ainsi un signal de sortie incluant des signaux de tous ou de la plupart des capteurs suggère qu'un grand nombre de capteurs ne sont pas recouverts d'une couche de cellules endothéliales.

La demande DE-A-101 03 503 divulgue un stent comprenant des électrodes pour mesurer l'impédance de tissu en contact du stent où chaque ensemble d'électrodes est associé à un multiplexeur contrôlé par un circuit de commande. L'implantation de tels multiplexeurs complexifie la structure du stent.

On connaît des demandes US-A-2011/0054583 et US-A-2012/0190989 une topologie matricielle de capteurs dans un stent. Une telle topologie permet un adressage des capteurs par ligne et par colonne, permettant une activation des capteurs de manière reconfigurable et indépendante les uns des autres.

Enfin, la demande WO-A-2011/121581 décrit un dispositif médical implantable capable de répondre à un champ électromagnétique d'interrogation émis par un dispositif d'interrogation distant. Le dispositif médical implantable est muni d'une pluralité de modulateurs constitués par des puces RFID (de l'anglais « Radio-Frequency IDentification » ou radio-identification). Les puces RFID sont adaptées pour que le dispositif médical implantable réponde à un champ électromagnétique d'interrogation selon une modulation générant un code d'identification respectif unique.

L'utilisation de puces RFID comme capteurs dans le dispositif médical limite cependant le nombre de capteurs dont il peut être muni. La multiplication des puces RFID augmente en effet d'autant le prix du dispositif médical. Ces puces RFID ne peuvent, en outre, être utilisées que comme capteurs d'impédance. Par ailleurs, selon ce document, le dispositif médical doit être au moins en partie en un matériau métallique ayant une bonne conduction électrique. Enfin, selon ce document, les puces RFID doivent être implantées dans la structure même du dispositif médical implantable, rendant la réalisation de celui-ci particulièrement complexe.

Il est également connu de WO-A-01/37 726 ou US-B-6 206 835, des dispositifs médicaux implantables. Ces dispositifs médicaux comportent une structure implantable dans le corps pour assister la réalisation d'une fonction vitale dans le corps. Un ou plusieurs capteurs sont associés à cette structure implantable, qui permet(tent) de mesurer un paramètre associé à la structure. Enfin, ces dispositifs médicaux comportent un circuit de communication couplé au(x) capteur(s) pour délivrer un signal fonction du paramètre mesuré et pour transmettre ce signal à un dispositif de réception, à l'extérieur du corps, de manière non invasive.

L'invention a pour objectif de pallier les problèmes évoqués plus haut. Notamment, l'invention a pour objectif de proposer un dispositif médical de structure simple et donc de coût limité, permettant de distinguer les grandeurs mesurées par différents capteurs dont est muni le dispositif médical. Dans un mode de réalisation préféré, le dispositif médical est implantable dans le corps du patient et configuré pour permettre de déterminer, sans intrusion dans le corps du patient, s'il est correctement implanté.

L'invention est définie par les revendications jointes et, de manière générale, concerne un dispositif médical comprenant un circuit électrique de mesure, dans lequel sont branchés au moins deux capteurs à impédance variable en fonction d'une grandeur physique captée, une source d'énergie électrique pour alimenter le circuit électrique de mesure, une antenne pour émettre un champ électromagnétique fonction de l'impédance du circuit électrique de mesure, chacun des capteurs étant associé à un interrupteur pour couper l'alimentation en courant du capteur dans ledit circuit de mesure, le dispositif médical comprenant en outre un système de commande des interrupteurs pour successivement commander l'ouverture ou la fermeture des interrupteurs, selon des configurations déterminées.

Ainsi, selon l'invention, le dispositif médical est muni de tout type de capteurs à impédance variable reliés ensemble dans un circuit dit de mesure. Un système de commande permet de couper l'alimentation en courant des différents capteurs selon des configurations prédéterminées, de sorte que le champ électromagnétique émis par le dispositif médical correspond à la configuration du circuit de mesure. En réalisant des mesures successives, correspondant à des configurations linéairement indépendantes - par exemple un capteur mis hors circuit à la fois ou tous les capteurs mis hors circuit à la fois sauf un - il est très facilement possible d'obtenir une information qualitative sur les valeurs mesurées par chacun des capteurs du dispositif médical, disposés à des emplacements connus sur le dispositif médical.

Par « mettre hors circuit un capteur », on entend par la suite créer une configuration du circuit telle que le courant traversant le capteur est nul, les autres capteurs pouvant être alimentés en courant. Le capteur est par exemple mis hors circuit en étant court-circuité à proprement parler ou mis hors circuit par ouverture du circuit du capteur, c'est-à-dire que le capteur est déconnecté de son circuit. En d'autres termes, « mettre hors circuit un capteur », dans les deux cas, signifie ici couper l'alimentation en courant de ce capteur.

De préférence, le dispositif médical comporte une ou plusieurs des caractéristiques suivantes, prises seules ou en combinaison :
- le dispositif médical est implantable dans le corps humain ou applicable sur le corps humain ;
- chaque interrupteur est formé par un ou des transistors, notamment un ou des transistors à effet de champ FET, plus particulièrement un ou des transistors à effet de champ à grille métal-oxyde MOS-FET, à enrichissement ou à appauvrissement, à canal N ou à canal P, un ou des MEMS, ou un ou des interrupteurs mécaniques ;
- le système de commande des interrupteurs comporte un circuit de commande, alimenté par la source d'énergie électrique, et conformé, de préférence, pour commander successivement l'ouverture ou la fermeture des différents interrupteurs, les uns après les autres ;

- le système de commande comporte des composants implantés directement dans le circuit de mesure, de préférence pour commander successivement l'ouverture ou la fermeture des différents interrupteurs, les uns après les autres ;
- le système de commande comporte des modules ayant la même structure, disposés en série les uns des autres, chacun commandant un interrupteur associé ;
- chaque module comporte un circuit RC série dont la charge ou la décharge provoque la fermeture ou l'ouverture de l'interrupteur associé ;
- la charge ou la décharge d'un circuit RC série d'un module engendre la charge ou la décharge du circuit RC série du module suivant ;
- chaque module comporte :
   ∘ un premier transistor dont la source est reliée à l'entrée du module, la grille à sa sortie et le drain à la grille de l'interrupteur associé,
   ∘ un premier inverseur dont l'entrée est reliée à celle du module et la sortie à une première borne de la résistance du circuit RC série, la deuxième borne de la résistance étant reliée à une première borne de la capacité, la deuxième borne de cette dernière étant reliée à la masse,
   ∘ un deuxième inverseur dont l'entrée est reliée à la deuxième borne de la résistance et la sortie à celle du module, laquelle sortie constitue l'entrée du module suivant.
- chaque module comporte :
   ∘ un premier transistor dont la grille est reliée à l'entrée du module et le drain à la borne d'alimentation positive,
   ∘ un deuxième transistor dont la source est reliée à celle du premier transistor, la grille à la sortie du module et le drain à une première borne du capteur à impédance variable dont la deuxième borne est reliée à la masse,
   ∘ une diode dont l'anode est reliée à la source du premier transistor et la cathode à une première borne de la résistance du circuit RC série, la deuxième borne de cette dernière étant reliée à la sortie du module, la capacité du circuit RC série étant connectée entre la sortie du module et la masse.
- chaque ensemble d'un interrupteur et d'un capteur est monté en série et les ensembles d'un interrupteur et d'un capteur sont montés en parallèle les uns par rapport aux autres ;
- chaque ensemble d'un interrupteur et d'un capteur est monté en parallèle et les ensembles d'un interrupteur et d'un capteur sont montés en série les uns des autres ; la source d'énergie électrique comporte une surface conductrice de courant du dispositif médical, adaptée à induire un courant électrique sous l'effet d'un champ électromagnétique ;
- au moins un des capteurs est disposé sur une surface du dispositif médical, destinée à être en contact avec une partie du corps sur laquelle le dispositif est appliqué ou dans laquelle le dispositif est implantable ;
- l'antenne est formée par une partie au moins du dispositif médical ;
- le circuit de mesure comporte une pluralité d'impédances fixes, associées chacune à un interrupteur dont l'ouverture et la fermeture sont commandées par le système de commande ;le dispositif médical est implantable dans le corps humain et est choisi parmi le groupe comprenant :
   - un tuteur vasculaire ou stent, au moins un capteur étant de préférence disposé sur une surface abluminale du tuteur vasculaire,
   - une valve cardiaque,
   - un stimulateur cardiaque,
   - un implant cochléaire,
   - un implant pour la gorge,
   - un implant orthopédique,
   - un implant cérébral,
   - un implant rétinien,
   - un cathéter, ou
   - un tissu cellulaire ;
- chaque capteur est choisi parmi :
   - un capteur de cisaillement,
   - un capteur de pression,
   - un capteur d'impédance,
   - un capteur de dissipation de chaleur,
   - une jauge de contrainte, et
   - un capteur de débit, notamment du type à fil chaud ;
- le dispositif médical implantable est un tuteur vasculaire avec au moins un capteur d'impédance disposé sur une surface abluminale du tuteur vasculaire ;
- le dispositif médical forme un conduit traversant, le dispositif médical comportant, deux capteurs de pression disposés à chacune des extrémités du conduit traversant, dans le conduit traversant ;
- le dispositif médical comprend entre deux capteurs chacun associé à un interrupteur, un élément d'impédance fixe et connue, associé à un interrupteur, un tel élément d'impédance fixe et connue étant de préférence prévu entre chacun des ensembles d'un capteur associé à un interrupteur ;
- le dispositif médical est un tuteur vasculaire maillé, au moins un capteur, de préférence tous les capteurs, étant disposé au milieu d'un côté d'une maille du tuteur vasculaire ;
- le dispositif médical comprend au moins un convertisseur analogique/numérique dont le signal de sortie commande l'antenne émettrice, éventuellement indirectement, notamment à travers une résistance variable ; et
- le dispositif médical comprend une pluralité de lignes de mesure, les lignes de mesure s'étendant sur le dispositif médical, notamment sur le tuteur vasculaire, selon des hélices coaxiales.

L'invention se rapporte également à un système médical comprenant un dispositif médical tel que décrit ci-avant dans toutes ses combinaisons et une unité de réception d'informations depuis le dispositif médical, comprenant des moyens pour capter le champ électromagnétique émis par l'antenne du dispositif médical.

Le système médical peut comprendre en outre une unité d'interrogation du dispositif médical, de préférence confondue avec l'unité de réception d'information, comprenant de préférence des moyens pour émettre un champ électromagnétique adapté à créer un courant induit dans le circuit de mesure du dispositif médical.

Le système médical peut comprendre un comparateur destiné à comparer un identifiant émis par l'unité d'interrogation, avec un code binaire associé à une combinaison donnée d'impédances fixes du circuit de mesure du dispositif médical.

Le système médical peut encore comprendre une unité de traitement des informations reçues par l'unité de réception, par exemple un ordinateur, l'unité de traitement des informations présentant de préférence un écran pour afficher en temps réel un modèle du dispositif médical sur lequel sont portées des informations relatives aux valeurs des mesures réalisées à l'aide des capteurs.

L'invention concerne également un procédé d'interrogation d'un dispositif médical tel que décrit ci-avant dans toutes ses combinaisons, notamment dans un système médical tel que décrit ci-avant dans toutes ses combinaisons, comprenant les étapes consistant à :
- alimenter le circuit de mesure du dispositif médical,
- activer le système de commande pour qu'il commande successivement l'ouverture ou la fermeture de chacun des interrupteurs, selon des configurations déterminées, et
- mesurer le champ électromagnétique émis par l'antenne du dispositif médical.

Le procédé peut comprendre une étape d'identification du dispositif médical.

Le procédé peut aussi comporter une étape de calibration avant chaque mesure ou avant certaines mesures du champ magnétique émis par l'antenne du dispositif médical, correspondant au champ magnétique émis par l'antenne en fonction du courant traversant les impédances et/ou les éléments d'impédance fixe et connue.

Les figures annexées feront bien comprendre comment l'invention peut être réalisée. Sur ces figures, des références identiques désignent des éléments semblables.
La figure 1 représente schématiquement un premier exemple de système médical comprenant un dispositif médical.
La figure 2 représente schématiquement un détail du circuit électrique du stent de la figure 1.
La figure 3 représente schématiquement un second exemple de système médical comprenant un dispositif médical.
La figure 4 représente schématiquement un troisième exemple de système médical comprenant un dispositif médical.
La figure 5 montre schématiquement un détail du dispositif médical de la figure 4.
La figure 6 représente schématiquement un quatrième exemple de système médical comprenant un dispositif médical.
Les figures 7 et 8 sont des représentations schématiques de variantes des circuits électriques des figures 1 et 3.
Les figures 9 et 10 illustrent schématiquement une variante du circuit électrique des figures 4 et 5.
Les figures 11 et 12 illustrent deux exemples de circuits électriques présentant plusieurs lignes de mesure.
La figure 13 représente schématiquement en perspective un stent muni de capteurs.
La figure 14 est un ordinogramme d'un procédé d'interrogation d'un dispositif médical.
La figure 15 représente schématiquement un comparateur d'identifiant du dispositif médical.

Dans la suite de la description, les éléments identiques ou de fonction identique, portent le même signe de référence dans les différents modes de réalisation. À fin de concision de la présente description, ces éléments ne sont pas décrits en regard de chacun des modes de réalisation, seules les différences entre les modes de réalisation étant décrites.

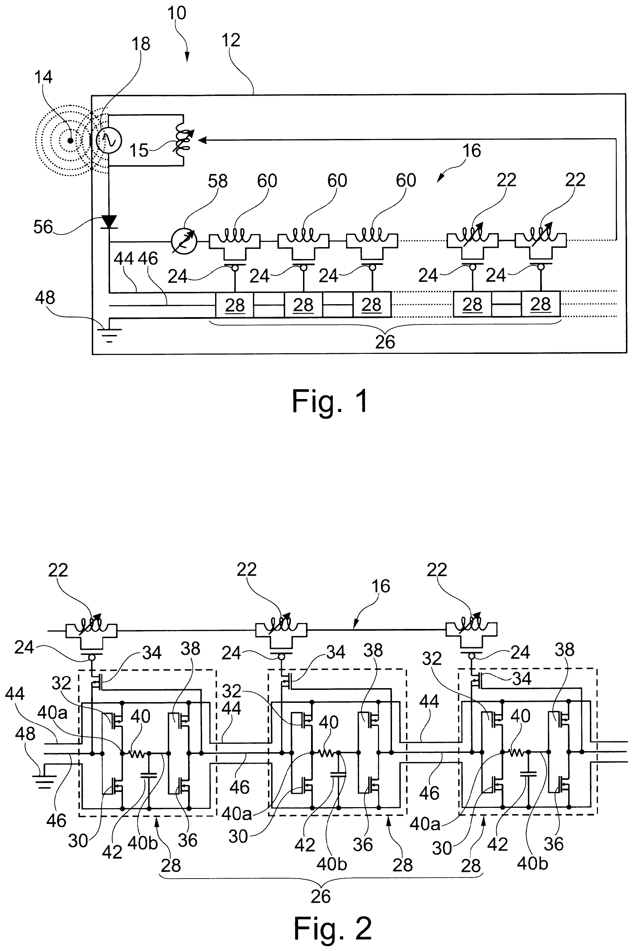

La figure 1 illustre schématiquement un système médical 10 comprenant un dispositif médical implantable 12 et une unité 14, ici unique, d'interrogation du dispositif médical 12 et de réception d'informations depuis ce même dispositif médical 12. Bien entendu, les unités d'interrogation et de réception d'informations peuvent, en variante, être distinctes. Le système médical 10 peut en outre comprendre une unité de traitement des informations reçues par l'unité de réception, par exemple un ordinateur.

Le dispositif médical implantable 12 comporte une impédance variable 15. La valeur de cette impédance variable 15 est commandée par une unité de commande non représentée, en fonction de l'impédance dans un circuit de mesure 16, reliant notamment les différents capteurs 22 du dispositif médical implantable. Le dispositif médical implantable 12 comporte encore une source d'énergie électrique, ici une source de courant électrique formée par le corps 18 du dispositif médical implantable 12. En effet, sous l'effet d'un champ électromagnétique émis par l'unité d'interrogation 14, le corps 18 du dispositif médical implantable 12 induit un courant. En variante, une antenne ou un induit séparé(e) et électriquement isolé(e) du corps 18 du dispositif médical implantable 12 peut également être prévu, notamment dans le cas où le dispositif médical implantable 12 n'est pas adapté, en tout ou partie, à avoir une fonction d'induit. Dans ce dernier cas notamment, une source d'énergie électrique pour le circuit de mesure peut comporter une surface conductrice de courant du dispositif médical implantable, adaptée à induire un courant électrique sous l'effet d'un champ électromagnétique. Une batterie ou pile électrique peut également être prévue comme source d'énergie électrique pour le dispositif médical implantable 12.

Le corps 18 du dispositif médical implantable 12 sert ici également d'antenne émettrice, pour émettre un champ électromagnétique vers l'extérieur du corps dans lequel le dispositif médical implantable est implanté. Par exemple, à intensité du courant induit constante de la source d'énergie électrique, l'intensité de ce champ dépend directement de l'impédance variable 15, fonction de l'impédance dans le circuit de mesure 16. Ainsi, l'intensité ou une norme du champ électromagnétique émis par le corps 18 du dispositif médical implantable 12 (ou plus généralement de l'antenne émettrice) est fonction de l'impédance du circuit de mesure 16. En variante, le dispositif médical implantable 12 peut comporter une antenne distincte du corps du dispositif médical implantable ou l'antenne peut être formée par une partie au moins du dispositif médical implantable.

Le dispositif médical implantable 12 est par exemple un stent. Le stent est un dispositif métallique tubulaire, de préférence maillé, glissé dans une cavité naturelle humaine (ou animale) pour la maintenir ouverte, comme décrit précédemment en introduction. Le stent peut par exemple être en alliage métallique ou en matériau polymère, mais d'autres matériaux sont également envisageables.

Le dispositif médical implantable 12 est muni de capteurs 22 à impédance variable en fonction de la grandeur physique qu'ils captent. Par grandeur physique, on entend ici toute propriété de la science de la nature qui peut être quantifiée par la mesure ou le calcul, et dont les différentes valeurs possibles s'expriment à l'aide d'un nombre réel quelconque ou d'un nombre complexe. Une grandeur physique inclut donc, par exemple, une longueur, un courant électrique, une tension, une impédance, une concentration en un élément chimique ou même la présence et/ou la concentration d'un élément biologique ou biochimique.

Les capteurs 22 sont répartis sur la surface du dispositif médical implantable. Dans le cas particulier du stent décrit ici, les capteurs 22 peuvent notamment être répartis :
- uniquement sur la surface « abluminale » du corps du stent, c'est-à-dire la surface opposée à la lumière à travers le stent, destinée à être en contact avec la paroi de la cavité à maintenir ouverte mais pas sur la surface luminale ; ou
- uniquement sur la surface luminale mais pas sur la surface abluminale ; ou
- à la fois sur les surfaces luminale et abluminale ; et
- sur les surfaces reliant les surfaces luminale et abluminale.

Les capteurs peuvent être enduits d'un agent actif, par exemple pour limiter l'hyperplasie des tissus en contact avec le dispositif médical implantable, notamment quand ils sont positionnés sur la surface abluminale d'un stent ou plus généralement sur la surface extérieure d'un dispositif médical implantable destiné à être en contact avec la paroi de la cavité dans laquelle le dispositif médical est implantable.

Il est à noter que le positionnement d'un seul capteur, notamment d'un capteur de pression, sur la surface abluminale d'un stent, ou plus généralement sur la surface extérieure d'un dispositif médical implantable permet déjà d'avoir une information relative au mauvais positionnement du stent ou du dispositif médical implantable dans la cavité. Si la pression mesurée est faible (i.e. inférieure à une pression seuil), il est probable que le capteur n'est pas en contact avec une paroi de la cavité, mais plutôt avec du sang, par exemple. Dans le cas où deux capteurs ou plus sont disposés sur la surface abluminale ou extérieure, l'information peut être obtenue avec plus de précision en comparant les valeurs mesurées par les capteurs les unes avec les autres.

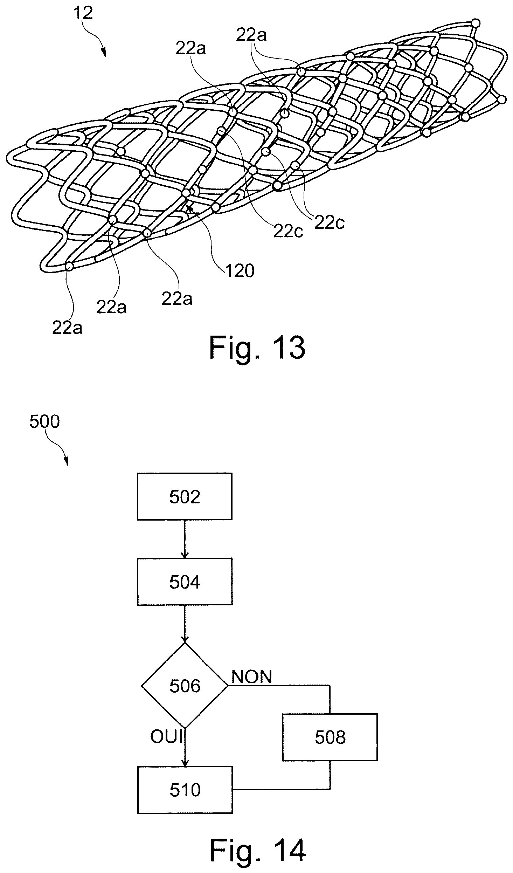

De préférence, les capteurs sont disposés aux emplacements du dispositif médical implantable, notamment d'un stent, subissant les déformations les moins importantes lors de la mise en place du dispositif médical implantable, ceci afin d'éviter d'endommager les capteurs. Ainsi, bien que la figure 13 montre un stent 12 avec des capteurs 22a fixés aux sommets des mailles 120 du stent 12 et des capteurs 22c fixés au milieu des côtés des mailles du stent, il est préféré que tous les stents 22c soient fixés au centre de côtés des mailles 120 du stent 12. Les capteurs 22a, 22c peuvent être disposés sur la face interne ou sur la face externe du stent 12. Cependant, il est particulièrement intéressant de placer un capteur de pression au voisinage de chaque extrémité du stent 12, sur la face interne du stent. Ainsi, une différence de pression entre les valeurs mesurées par ces deux capteurs peut être déterminée qui permet d'identifier l'apparition d'un bouchon à l'intérieur du stent.

Chacun des capteurs peut notamment être choisi parmi :
- un capteur de cisaillement,
- un capteur de pression,
- un capteur d'impédance,
- un capteur de dissipation de chaleur,
- une jauge de contrainte, et
- un capteur de débit du type « capteur à fil chaud » (de l'anglais « hot wire sensor »).

Les capteurs 22 sont des capteurs à impédance variable, c'est-à-dire des capteurs dont l'impédance varie en fonction de l'amplitude ou de l'intensité de la grandeur physique captée. Par suite, en cas de variation de l'amplitude de la grandeur physique captée par un capteur du dispositif médical implantable 12, l'impédance de ce capteur varie dans le circuit de mesure 16, de sorte que, en l'absence de toute autre variation dans le circuit de mesure 16, l'impédance du circuit de mesure 16 varie également.

Comme illustré, chaque capteur 22 est associé à un interrupteur 24 adapté à mettre hors circuit, en l'occurrence court-circuiter, le capteur 22 auquel il est associé. Ici, ceci est réalisé en montant l'interrupteur 24 en dérivation (ou en parallèle) du capteur 22 auquel il est associé. Les capteurs 22 sont ici montés en série dans le circuit de mesure 16. Pour des raisons de facilité de réalisation et de miniaturisation, chaque interrupteur est ici réalisé par un transistor 24, en l'espèce un transistor MOS-FET au silicium, plus précisément un transistor MOS-FET à canal P, à appauvrissement (ou p-MOS). Dans d'autres modes de réalisations, chaque interrupteur ou certains interrupteurs peuvent être réalisés par un autre type de transistor, notamment par un transistor FET, un transistor MOS-FET à enrichissement, notamment un transistor MOS-FET à canal N à enrichissement, par un MEMS (de l'anglais « Microelectromechanical system », ou par un interrupteur mécanique.

La figure 1 illustre en outre un système 26 de commande des interrupteurs 24, adapté à successivement commander l'ouverture ou la fermeture des interrupteurs 24 selon des configurations déterminées. Le système de commande 26 comporte des modules de commande 28 disposés en série les uns des autres, chaque module de commande 28 étant adapté à commander l'ouverture ou la fermeture de l'interrupteur 24 auquel il est associé.

Dans le cas d'espèce, le système de commande 26 est conformé pour normalement maintenir fermés les interrupteurs 24 et pour les ouvrir successivement puis pour les refermer de sorte qu'à chaque instant un unique interrupteur 24 est ouvert.

Pour ce faire, chaque module de commande 28 est formé ici d'un circuit logique, réalisé au moyen de transistors 30, 32, 34, 36, 38, d'une résistance 40 et d'un condensateur 42. La résistance 40 et le condensateur 42 introduisent un temps de charge du condensateur 42 et un temps de décharge de ce même condensateur 42 dans le circuit logique. Pendant ces temps de charge et décharge, le module de commande 28 commande l'ouverture de l'interrupteur 24 associé. L'interrupteur 24 est maintenu fermé le reste de temps, court-circuitant alors le capteur 22 associé.

Plus précisément, et comme représenté sur la figure 2, ici, chaque module de commande 28 est réalisé au moyen de trois transistors 32, 34, 38 à canal P et deux transistors 30, 36 à canal N, comme suit (seules les liaisons ci-dessous sont réalisées) :
- des première et deuxième branches 44, 46 du circuit de mesure 16 sont branchées en parallèle, la première branche 44 étant la borne d'alimentation positive et la deuxième branche 46 étant l'entrée du circuit de mesure 16 qui est alimentée par un circuit de départ, non représenté sur les figures, configuré pour générer une impulsion de tension en forme de créneau pendant un certain intervalle temporel;
- la grille du premier transistor 30 et la grille du deuxième transistor 32 sont reliées ensemble, ainsi qu'à la source du troisième transistor 34 et à la deuxième branche 46 du module de commande 28 précédant, les deux transistors 30 et 32 formant un premier inverseur ;
- la grille du quatrième transistor 36 et la grille du cinquième transistor 38 sont reliées ensemble ainsi qu'à une borne de la résistance 40 et à une borne du condensateur 42, les deux transistors 36 et 38 formant un deuxième inverseur ;
- la source du premier transistor 30, la source du quatrième transistor 36 et une borne du condensateur 42 sont reliées à la masse 48 ;
- l'autre borne de la résistance 40, qui n'est pas reliée au condensateur 42, est reliée au drain du premier transistor 30 et au drain du deuxième transistor 32 ;
- le drain du quatrième transistor 36 et le drain du cinquième transistor 38 sont reliés ensemble à la deuxième branche 46 du module de commande 28 suivant, ainsi que la grille du troisième transistor 34 ;
- la source du deuxième transistor 32 et la source du cinquième transistor 38 sont reliés ensemble à la première branche 44 du module de commande 28 précédant ;
- le drain du troisième transistor 34 est relié à la grille du transistor 24 ayant une fonction d'interrupteur pour court-circuiter le capteur 22.

Lorsque la tension appliquée à l'entrée du premier inverseur est proche de zéro, donc inférieure à la tension de seuil des transistors 30 et 32, le transistor 32 de type PMOS devient passant, chargeant la capacité 42. A la fin de la charge de cette dernière, la tension à l'entrée du deuxième inverseur est supérieure à la tension de seuil des transistors 36 et 38, rendant le transistor 36 de type NMOS passant. Une tension proche de zéro est transmise à la sortie du deuxième inverseur connectée à la grille du transistor 34 de type PMOS. Celui-ci devient alors passant, transmettant une tension proche de zéro à la grille de l'interrupteur 24 de type PMOS, ce qui provoque sa fermeture. Tant qu'une tension proche de zéro est appliquée à l'entrée du premier inverseur, l'interrupteur 24 est maintenu fermé.

Lorsqu'on applique à l'entrée du premier inverseur une tension supérieure à la tension de seuil des transistors 30 et 32, le transistor 30 de type NMOS devient passant, transmettant à sa sortie le potentiel de la masse, ce qui provoque la décharge de la capacité 42. Lors de cette décharge, la tension à l'entrée du deuxième inverseur diminue jusqu'à devenir inférieure à la tension de seuil des transistors 36 et 38, bloquant le transistor 36 et activant le transistor 38. Celui-ci transmet alors à la sortie du deuxième inverseur un potentiel supérieur à la tension de seuil du transistor 34, provoquant son blocage. Par conséquent, l'interrupteur 24 s'ouvre. Ainsi, en appliquant une tension positive importante à l'entrée du premier inverseur du premier module de commande 28, on engendre l'ouverture de l'interrupteur 24 qui lui est connecté, puis l'ouverture successive des interrupteurs 24 connectés aux modules de commande 28 qui suivent. Le circuit de départ, non représenté, alimentant l'entrée 46 du premier module est configuré pour générer une impulsion de tension en forme de créneau pendant un intervalle de temps τ = RC. Le front descendant de cette impulsion engendre la fermeture de l'interrupteur du premier module après un temps égal à τ. L'impulsion de tension se propage d'une entrée 46 à une autre, de façon que le front descendant de cette impulsion en entrée d'un module n corresponde au front montant de l'impulsion en entrée du module n+1. Ainsi, pour le cas d'espèce, lors de la propagation de l'impulsion, tous les interrupteurs sont fermés sauf un.

Avec un tel système de commande, la tension aux bornes du circuit de mesure 16, qui est égale à la somme des tensions aux bornes de chacun des capteurs montés en série dans le circuit de mesure, présente des pics successifs qui sont représentatifs de la tension aux bornes de chacun des capteurs. À chacun des pics successifs, représentatifs chacun de la tension aux bornes d'un capteur 22, correspond une intensité du champ électromagnétique émis par le corps 18 du dispositif médical implantable 12 ayant une fonction d'antenne émettrice.

Sur la figure 1, on constate la présence d'un redresseur 56 ainsi que d'un générateur de courant alternatif 58 dans le dispositif médical implantable 12. Ils permettent respectivement d'alimenter le circuit de commande 26 en courant continu et le circuit de mesure 16 avec un courant ayant une fréquence distincte de, notamment inférieure à, la fréquence du courant induit dans l'antenne 18. Ceci est peut-être utile car la fréquence du courant induit est fonction du champ électromagnétique émis par l'unité 14, laquelle fréquence est de préférence choisie pour que l'onde électromagnétique soit peu absorbée par les tissus traversés. L'utilisation d'une telle fréquence dans le circuit de mesure pourrait nuire à la précision des mesures effectuées.

Le circuit de mesure 16 est par ailleurs complété sur la figure 1, par une combinaison d'ensembles d'une impédance 60, fixe et connue, et d'un interrupteur 24, commandé par un module de commande 28, comme cela est le cas pour les interrupteurs 24 associés aux capteurs 22. Cette combinaison d'impédances connues permet d'identifier le dispositif médical implantable interrogé, par exemple en associant une combinaison d'impédances 60 unique et connue à chaque dispositif médical implantable 12. Ceci est particulièrement utile dans le cas où plusieurs tels dispositifs médicaux implantables ont été implantés dans le corps d'un même patient. Certains pics de champ électromagnétique mesurés sont alors utilisés pour identifier le dispositif médical implantable 12, les autres pics pour déterminer les valeurs mesurées par chacun des capteurs du dispositif médical implantable 12 identifié. Par exemple, les premiers pics de champ électromagnétique mesurés peuvent être utilisés pour identifier le dispositif médical implantable 12 et les pics ultérieurs pour déterminer les valeurs mesurées par chacun des capteurs du dispositif médical implantable 12 identifié. En outre, ces impédances étant connues, elles permettent également de calibrer le système médical 10. En d'autres termes, ces impédances connues permettent de quantifier plus précisément les valeurs mesurées par les différents capteurs des différents dispositifs médicaux implantables.

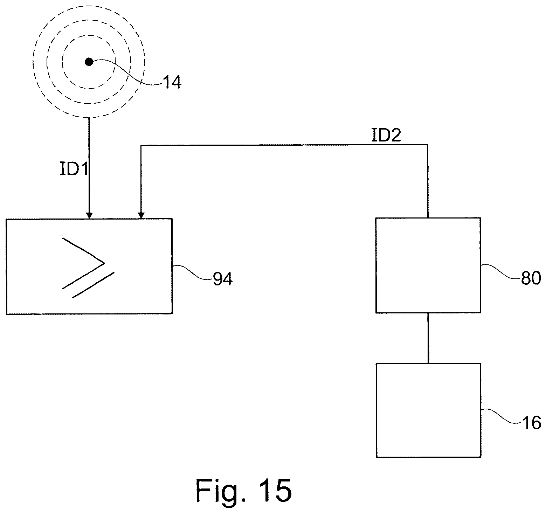

En variante, selon le mode de réalisation représenté partiellement et schématiquement à la figure 15, le dispositif médical peut comporter un comparateur 94 destiné à comparer un identifiant ID1 émis par l'unité d'interrogation 14, avec un code binaire ID2 associé à la combinaison d'impédances 60 présente sur le circuit électrique, ce code binaire étant issu par exemple de la sortie du convertisseur analogique/numérique 80, ou un identifiant enregistré dans une mémoire dans le stent. Le dispositif médical peut alors être configuré pour ne répondre à l'interrogation par l'unité d'interrogation que si la comparaison est positive. Avantageusement, l'identification peut être réalisée de manière itérative, l'unité d'interrogation n'émettant qu'une valeur de l'identification à la fois, chaque stent dont la valeur correspondant de l'identifiant ne correspondant pas étant désactivé - c'est-à-dire, ici, non alimenté électriquement - pour un temps permettant d'identifier le seul stent correspondant à l'identifiant unique et de mener à bien les mesures à l'aide des différents capteurs de ce dispositif médical.

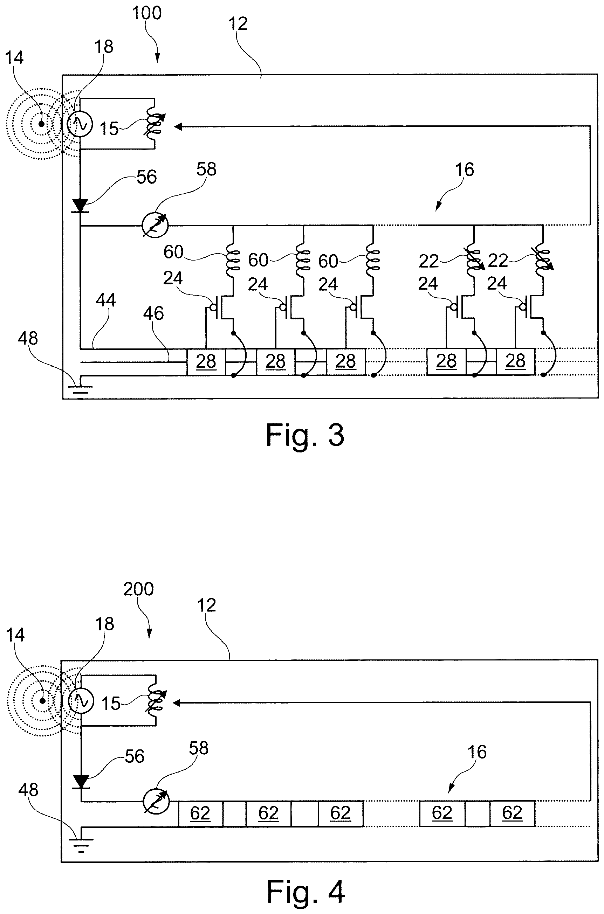

La figure 3 représente un deuxième exemple de système médical 100. Ce système médical est sensiblement identique à celui décrit précédemment. Cependant, dans ce mode de réalisation, dans le circuit de mesure 16 du dispositif médical implantable 12, les impédances 60 connues et les capteurs sont montés en série avec l'interrupteur 24 qui leur est associé, les ensembles formés d'une impédance 60 ou d'un capteur 22 et d'un interrupteur 24 étant montés en parallèle (ou en dérivation) les uns des autres. Par suite, les modules de commande 28 étant identiques à ceux décrits précédemment, le champ électromagnétique émis suite à la création d'un courant induit, correspond à la somme de toutes les impédances 60 et de tous les capteurs 22, moins un(e), chacune des impédances 60 et des capteurs 22 étant mis(e) hors circuit, en l'occurrence déconnecté(e), successivement.

En variante, bien entendu, on peut réaliser un module de commande 28 ayant un fonctionnement différent, qui commande la fermeture de l'interrupteur 24 durant un intervalle de temps seulement, l'interrupteur 24 étant ouvert le reste du temps. Un tel fonctionnement peut également être obtenu en conservant le module de commande 28 comme décrit précédemment et en remplaçant les transistors MOS-FET à appauvrissement mis en oeuvre en tant qu'interrupteurs 24 par des transistors MOS-FET à enrichissement.

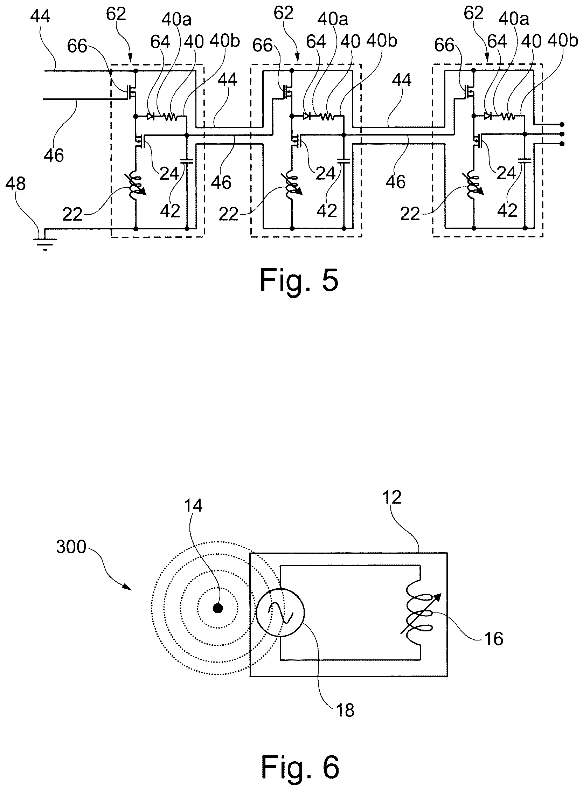

Les figures 4 et 5 illustrent un autre exemple de système médical 200. Selon cet exemple, la commande de l'interrupteur 24 de mise hors circuit du capteur 22 ou d'une impédance connue 60 est directement implantée dans un module 62 comprenant également l'impédance connue 60 ou le capteur 22, et l'interrupteur 24, ici réalisé par un transistor. Comme pour les autres exemples déjà décrits, une résistance 40 et un condensateur 42 sont mis en oeuvre pour commander l'interrupteur 24 de telle sorte qu'il mette hors circuit l'impédance 60 ou le capteur 22 sauf durant un intervalle de temps de charge du condensateur 42. La charge du condensateur 42 active le transistor 66 du module suivant, entraînant la charge du condensateur 42 correspondant. Une fois chargé, le condensateur 42 bloque le transistor 24 associé, provoquant la mise hors circuit de l'impédance 60 ou du capteur 22 qui lui est relié.

Ici, comme représenté à la figure 5, chaque module 62 est réalisé comme suit :
- les première et deuxième branches 44, 46 sont en parallèle ;
- une borne du capteur 22 ou de l'impédance 60 est reliée à la masse 48 ;
- l'autre borne du capteur 22 ou de l'impédance 60 est reliée au drain du transistor 24 ;
- la grille du deuxième transistor 66 est reliée à la deuxième branche 46 du module 62 précédant ;
- le drain du deuxième transistor 66 est relié à la première branche 44 des modules 62 précédant et suivant ;
- la source du deuxième transistor 66 est reliée à la source du transistor 24 et à une diode 64 ;
- l'autre borne de la diode 64, qui n'est pas reliée aux transistors 24, 66, est reliée à une borne d'une impédance 40 fixe ;
- l'autre borne de l'impédance 40, qui n'est pas reliée à la diode 64, est reliée à la grille du transistor 24, à une borne d'un condensateur 42, relié par son autre borne à la masse 48, et à la deuxième branche 46 du module 62 suivant.

Comme pour les exemples précédents, du fait de la configuration des modules 62, chaque capteur 22 et impédance 66 est successivement relié(e) à l'antenne 18 pour être alimenté(e), les autres capteurs 22 et impédances 66 étant pour leur part déconnectés.

Enfin, la figure 6 représente un quatrième exemple de mode de réalisation d'un système médical 300. Ce système médical 300 se distingue du précédent exemple 200, en ce que le circuit de mesure 16 est directement relié à l'antenne 18 pour l'émission d'un champ électromagnétique, sans l'intermédiaire d'une impédance variable distincte (le circuit de mesure 16 ayant lui-même une impédance variable) et d'une unité de commande de cette impédance variable en fonction de l'impédance du circuit de mesure 16. Le circuit électrique sur le dispositif médical 12 est alors particulièrement simplifié.

Bien entendu, on peut imaginer une structure où le circuit de mesure 16 est relié directement à l'antenne, le dispositif médical implantable comprenant également un circuit de commande associé à ce circuit de mesure et comme décrit par exemple en regard des figures 2 et 3.

En pratique, dans les modes de réalisation décrits précédemment, chaque module peut notamment être réalisé sous la forme suivante. Deux électrodes de mesures, par exemple de 60 × 60 µm², réalisées en un matériau conducteur électrique, par exemple en matériau polymère ou en alliage métallique, de préférence biocompatible, sont déposées sur un substrat polymérique biocompatible, isolant électrique (par exemple du parylène). Les composants électriques du système de commande et l'interrupteur sont implantés dans le substrat polymérique.

Les systèmes médicaux décrits précédemment permettent de mettre en oeuvre un procédé d'interrogation 500 du dispositif médical implantable 12, tel que représenté par l'ordinogramme de la figure 14.

Ce procédé 500 comporte une première étape 502 consistant à alimenter le circuit de mesure 16. De préférence, cette alimentation est réalisée par un courant induit dans une antenne ou dans le corps du dispositif médical implantable 12 quand celui-ci est conformé pour générer un courant induit. Ceci permet d'alimenter le circuit de mesure 16 uniquement quand une mesure est réalisée.

Le procédé 500 se poursuit par une étape 504 consistant à activer le système de commande du dispositif médical implantable pour qu'il commande successivement l'ouverture ou la fermeture de chacun des interrupteurs du dispositif médical implantable, selon des configurations déterminées. Il est à noter ici que dans le cadre des exemples décrits en regard des figures, cette activation est réalisée simultanément à l'alimentation du circuit de mesure 16, par induction, en réponse à l'émission d'un champ électromagnétique par le dispositif d'interrogation. Le procédé 500 comporte alors une étape 506 d'identification du dispositif médical interrogé. Cette étape peut, en variante, être réalisée avant d'alimenter électriquement le circuit de mesure.

L'identification peut être réalisée soit dans le dispositif médical lui-même, quand celui-ci est muni d'un comparateur pour comparer un signal d'identification émis par l'unité d'interrogation avec un identifiant unique du dispositif médical. Comme indiqué précédemment, cet identifiant peut prendre la forme d'une combinaison d'impédances connues dans le dispositif médical et/ou dans chaque ligne de mesure du dispositif médical. L'identification peut être réalisée de manière itérative, l'unité d'interrogation n'émettant qu'un digit de l'identifiant à la fois, chacun des dispositifs médicaux dont l'identifiant ne correspond pas à ce digit étant temporairement désactivé (c'est-à-dire, dans l'exemple étudié, non alimenté électriquement).

En variante, l'identification est réalisée dans l'unité de traitement, les signaux émis par l'antenne 18 étant interprétés par l'unité de traitement pour déterminer la combinaison des impédances du dispositif médical et/ou de la ligne de mesure interrogée. Une unité de traitement peut être mise en oeuvre pour déterminer la valeur mesurée par chaque capteur et le dispositif médical implantable qui a répondu à l'interrogation, notamment si les configurations commandées du circuit de mesure sont plus complexes.

Pour ce faire, l'unité de traitement peut notamment être adaptée à conduire des analyses de Fourier des signaux mesurés de champs électromagnétiques émis par l'antenne du dispositif médical implantable, à comparer les signaux reçus (éventuellement traités) à des signaux mesurés précédemment et à en déduire les valeurs mesurées par les différents capteurs du dispositif médical implantable, une localisation pouvant être déterminée pour chacune des valeurs mesurées

Si l'identification est négative, le dispositif médical est temporairement désactivé, à l'étape 508.

Si l'identification est positive, le procédé 500 se poursuit alors par une étape 510 consistant à mesurer le champ électromagnétique émis par l'antenne du dispositif médical implantable. Cette mesure est réalisée durant un temps assez long pour que le système de commande ait pu commander un nombre assez important de configurations différentes du circuit de mesure pour que la mesure permette de déterminer la valeur mesurée par chacun des capteurs 22 du dispositif médical implantable 12. Durant toute l'étape de mesure, l'antenne 14 émet de préférence un champ électromagnétique constant pour maintenir l'alimentation du circuit de mesure 16 et l'activation du système de commande 26.

De préférence, chaque configuration correspond au cas où tous les capteurs ou impédances du circuit de mesure sont mis(es) hors circuit, sauf un(e). Ainsi, à partir du champ électromagnétique mesuré, il est possible de déterminer tout d'abord le dispositif médical implantable qui a répondu à l'interrogation. En effet, les premiers pics mesurés dans le champ électromagnétique émis par l'antenne correspondent à des impédances fixes, dont la combinaison permet d'identifier le dispositif médical implantable. Ces champs magnétiques mesurés peuvent également permettre de calibrer le système puisque les champs magnétiques mesurés correspondent à des impédances du circuit de mesure connues. Enfin, les champs magnétiques ultérieurs permettent de déterminer les valeurs mesurées par chacun des capteurs répartis sur le dispositif médical implantable.

Lorsque des éléments 60A sont prévus entre les capteurs 22, les champs magnétiques émis correspondant peuvent être mis en oeuvre pour calibrer le signal émis qui suit et/ou qui précède, lequel provient d'une mesure par un capteur 22.

Une fois l'interrogation de tous les capteurs 22 du dispositif médical 12 réalisée, l'alimentation électrique du circuit électrique est interrompue et le circuit électrique du dispositif médical 12 est désactivé.

Il est à noter ici que le procédé décrit peut être mis en oeuvre avec tout type de capteur à impédance variable en fonction de la grandeur physique qu'il détecte. Il est à noter également que les capteurs répartis sur le dispositif médical implantable peuvent être de natures différentes, c'est-à-dire qu'ils peuvent capter des grandeurs physiques différentes.

Le procédé décrit ci-avant peut notamment être mis en oeuvre pour déterminer si le dispositif médical implantable est convenablement implanté (c'est-à-dire positionné) dans la cavité naturelle qu'il est censé maintenir ouverte, notamment, s'il est bien en contact avec la paroi de la cavité. En effet, l'effet d'un stent, par exemple mais cela est vrai pour la plupart des dispositifs médicaux implantables, est nettement réduit si celui-ci n'est pas en appui sur la paroi de la cavité (notamment de la veine ou de l'artère) dans laquelle il est introduit.

Par exemple, en plaçant des capteurs de pression sur la surface abluminale du stent, c'est-à-dire sur la surface opposée à la lumière à travers le stent, celle qui est destinée à être en contact avec la paroi de la cavité dans laquelle le dispositif médical implantable est reçu, le procédé décrit précédemment permet de déterminer si chacun de ces capteurs est en contact avec la paroi, puisqu'il permet de déterminer la pression mesurée par chacun des capteurs. Bien entendu, cette fonction de détermination de la position convenable du stent peut être combinée, c'est-à-dire que des capteurs, par exemple de pression, peuvent être disposés sur la surface abluminale du stent et des capteurs, éventuellement d'une autre grandeur physique, peuvent être disposés sur la surface luminale du stent.

En variante, des capteurs d'une même grandeur physique sont répartis sur la surface abluminale et sur la surface luminale, sensiblement à la même position sur le stent ou du dispositif médical implantable. En d'autres termes, des capteurs d'une même grandeur physique sont disposés au même point du stent, de part et d'autre du corps du stent. La comparaison des valeurs mesurées par chacun de ces couples de stent permet également d'avoir des indices d'une mauvaise position du stent dans la cavité. Notamment si le capteur sur la surface abluminale, qui devrait donc être en contact avec une paroi, mesure une valeur sensiblement identique au capteur sur la surface luminale, qui est en contact avec le sang, il est probable que le capteur sur la surface abluminale est en fait en contact avec du sang également, non avec une paroi. Il est donc probable que le stent est mal positionné dans la cavité.

Bien entendu, le procédé décrit précédemment peut permettre d'obtenir de nombreuses autres informations.

Notamment, il peut permettre de déterminer si un capteur disposé sur la surface luminale ou abluminale du stent ou, plus généralement, sur une surface d'un dispositif médical implantable, notamment sur une surface du dispositif médical implantable en contact avec une paroi de la cavité dans laquelle le dispositif médical est implanté ou sur une surface du dispositif médical implantable destinée à être en contact avec le sang, est ou non recouvert de tissu endothélial ou musculaire-lisse.

Il peut également permettre de déterminer la constitution du tissu recouvrant les capteurs répartis sur le dispositif médical implantable (notamment sur la surface luminale ou sur la surface abluminale d'un stent) par exemple par spectroscopie d'impédance électrique (EIS, de l'anglais « Electrical Impédance Spectroscopy »), notamment en appliquant des courants de fréquences distinctes dans le circuit de mesure.

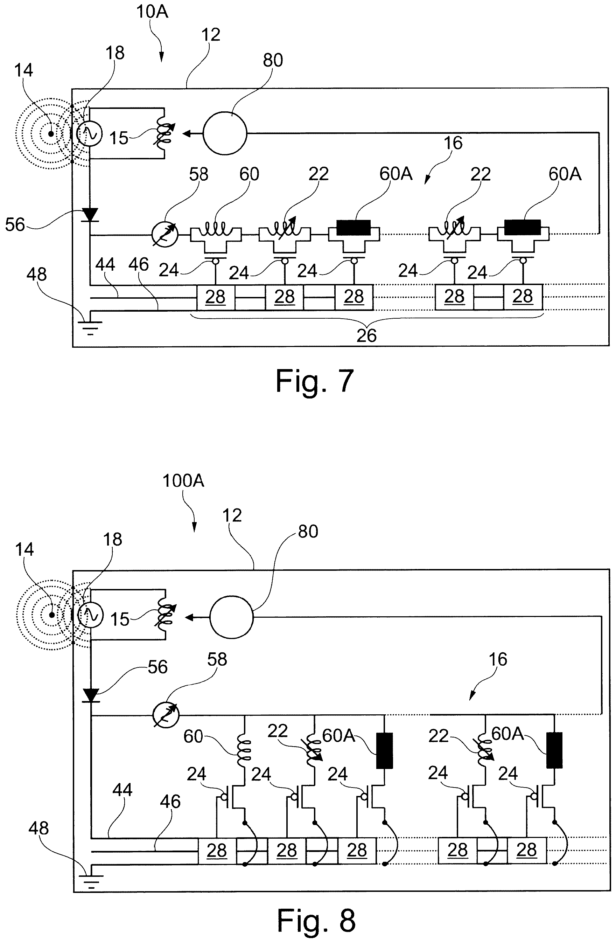

Le circuit électrique 10A de la figure 7 est une variante du circuit de la figure 1.

Ce circuit électrique 10A comporte tout d'abord un convertisseur 80 analogique/numérique situé entre le circuit électrique de mesure 16 et l'impédance variable 15 reliée à l'antenne 18 dans le circuit émetteur. Ce convertisseur 80 analogique/numérique, qui peut également être mis en oeuvre dans le circuit électrique 10 de la figure 1 permet d'accroitre la plage des signaux qui peuvent être émis par l'antenne 18. Ce convertisseur 80 permet également d'améliorer le rapport signal/bruit des mesures réalisées.

Par ailleurs, le circuit électrique 10A se distingue de celui de la figure 1 par la présence d'un élément 60A d'impédance fixe et connue, par exemple une résistance, entre les capteurs 22. Comme pour les impédances 60, l'élément 60A est associé à un interrupteur 24 commandé par un module de commande 28 dans un module 62A. L'impédance de cet élément 60A est de préférence choisie de telle sorte que les capteurs 22 ne puissent pas atteindre cette valeur d'impédance. Par exemple, l'impédance de l'élément 60A est inférieure à 90% de l'impédance minimale que peut atteindre un capteur 22 et/ou supérieure à 110% de l'impédance maximale que peut atteindre un capteur 22. Ainsi, entre deux signaux émis relatifs à une mesure par un capteur 22, un signal de transition, facilement identifiable, est émis. Il est plus aisé, ainsi, de distinguer entre deux mesures successives, séparées par un signal de forme attendue. Par ailleurs, la présence de cet élément 60A entre les capteurs 22 peut permettre de calibrer le capteur 22 qui suit et/ou celui qui précède. La mesure est donc plus précise. Bien sûr, d'autres configurations sont envisageables. Notamment, il peut être prévu un élément 60A en début de ligne uniquement, tous les n capteurs 22, n étant un entier naturel non nul, ou même une répartition irrégulière des éléments 60A sur la ligne de mesure.

La figure 8 représente une variante 100A du circuit électrique 100 de la figure 3, avec un convertisseur 80 analogique/numérique et des éléments 60A d'impédance connue et fixe, comme le circuit électrique 10A. Les avantages de ces modifications sont les mêmes que dans le cadre du circuit électrique 10A de la figure 7.

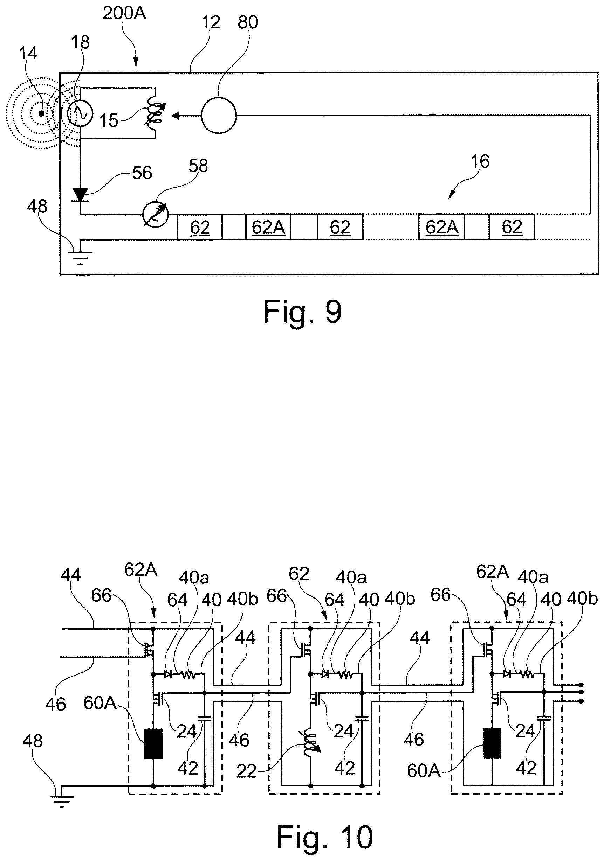

Il en va de même du circuit électrique 200A illustré par les figures 9 et 10 et obtenu en apportant les mêmes modifications au circuit électrique 200 des figures 4 et 5.

Il est à noter ici, que la présence des éléments 60A d'impédance connue et du convertisseur 80 analogique/numérique sont indépendantes. Des modes de réalisations peuvent être envisagés ne présentant pas l'un des deux parmi le convertisseur 80 analogique/numérique et les éléments 60A d'impédance connue.

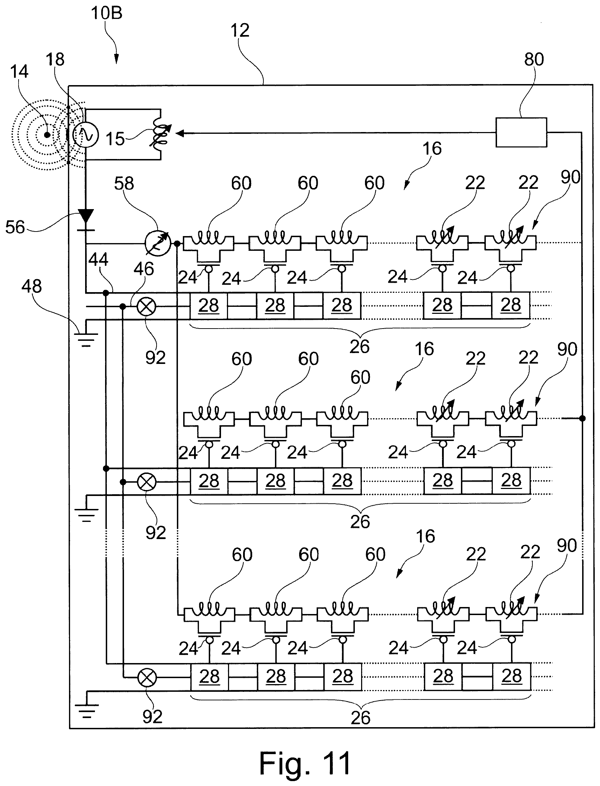

Sur la figure 11, on a représenté un schéma électrique d'un circuit électrique 10B variante du circuit électrique 10 de la figure 1. Ce circuit électrique 10B se distingue du circuit électrique 10 de la figure 1 en ce qu'il comporte plusieurs lignes de mesure 90, montées en parallèle, là où le circuit électrique 10 de la figure 1 ne comporte qu'une seule ligne. Ici, les lignes de mesure 90 sont identiques à la seule ligne représentée sur la figure 1. Le circuit de mesure 16 comporte cependant des sélecteurs de ligne 92, pour réaliser la mesure dans chacune des lignes indépendamment des autres lignes 90, notamment chaque ligne à la suite d'une autre. Les sélecteurs de ligne 92 peuvent prendre une forme sensiblement identique aux modules de commande 28, alimentant ainsi chaque ligne 90 en courant, successivement.

Sur l'exemple représenté, un convertisseur 80 analogique/numérique est également prévu entre les branches parallèles formées par les lignes de mesure 90 et l'impédance variable 15.

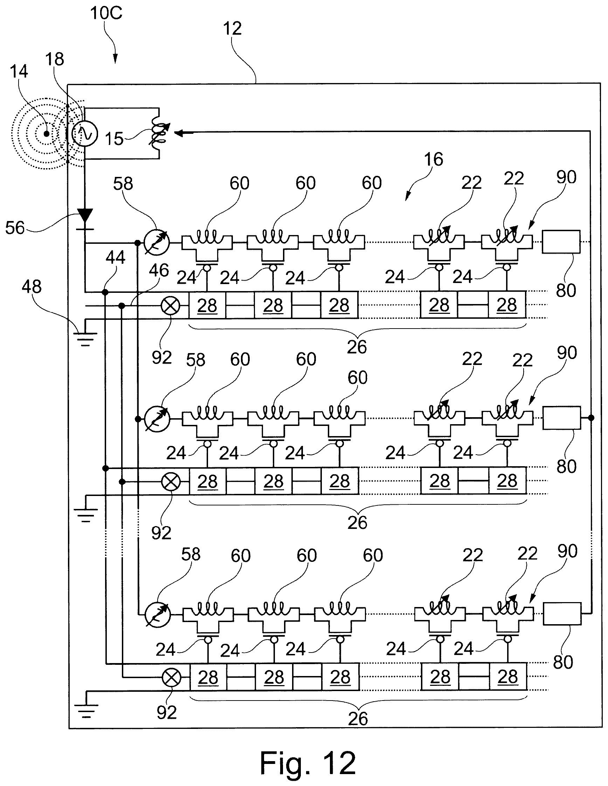

Le circuit électrique 10C illustré à la figure 12 est proche du circuit électrique 10B de la figure 11. Il se distingue essentiellement par la présence d'un convertisseur 80 analogique/numérique sur chacune des lignes de mesure 90 en parallèle.

Il est à noter qu'il est également possible de prévoir des circuits électriques à plusieurs lignes de mesures 90 sur la base des circuits électriques 100 et 200 des figures 3 et 4.

Il est également possible de prévoir des éléments 60A à impédance connue et fixe entre les capteurs sur chacune de ces lignes ou sur certaines lignes seulement. Il est encore possible d'identifier la ligne dont la mesure est réalisée à chaque instant en choisissant des combinaisons d'impédances 60 en début de ligne 90 uniques pour chaque ligne 90.

Dans le cas où le circuit électrique 10B, 10C comporte plusieurs lignes de mesure, il a été déterminé qu'il est particulièrement intéressant que les lignes de mesure s'étendent sur le dispositif médical 12, notamment sur le stent 12, en hélices coaxiales imbriquées. En d'autres termes, les lignes de mesure s'étendent parallèlement selon des hélices enroulées les unes autour des autres. En effet, ceci permet de minimiser la distance entre des capteurs de lignes différentes. Ceci est particulièrement intéressant car si un capteur 22 d'une ligne 90, voire toute la ligne 90 est défaillant(e), la ou les valeurs manquantes peuvent être mieux approximée(s) par les valeurs mesurées avec la ou les autres lignes de mesure, dont un ou plusieurs capteurs sont situés à proximité. Le dispositif 12 gagne ainsi en robustesse, ce qui est particulièrement intéressant lorsqu'il est implanté dans le corps d'un patient.

Il est à noter que les circuits électriques décrits permettent de déterminer pour chaque capteur des circuits électriques, la valeur qu'il a mesurée. La position des capteurs sur le dispositif médical, notamment sur le stent, étant connue, il est possible de déterminer un modèle représentant en temps réel, l'évolution des paramètres physiques mesurés sur le dispositif médical. Ainsi, un praticien peut obtenir une information en temps réel. Cette information peut notamment être relative au bon positionnement du dispositif médical, notamment du stent, dans une cavité du corps humain. La représentation des pressions mesurées par des capteurs de pression disposés sur la surface externe du dispositif médical, notamment du stent, peuvent permettre au praticien de déterminer si ce dispositif médical est correctement implanté ou non : une pression mesurée trop faible, par exemple, peut indiquer que le stent n'est pas en contact avec la paroi de la cavité le recevant.

L'unité de traitement du système médical décrit précédemment, comprenant par exemple une unité électronique de commande et un écran, ou un ordinateur, peut être adaptée à déterminer un modèle en temps réel, par exemple un modèle 3D, à partir des valeurs mesurées et à afficher le modèle sur l'écran. Les valeurs entre les points de mesure peuvent, dans ce cas, être approximées, notamment par convolution en fonction de la distance aux points de mesure les plus proches.

Divers signaux visuels et/ou acoustiques peuvent être émis par l'unité de traitement, dans le cas où au moins une valeur mesurée n'est pas conforme aux attentes. Les signaux visuels peuvent notamment permettre d'identifier sur le modèle représenté, les capteurs 22 pour lesquels les valeurs mesurées ne sont pas conformes.

En variante, l'unité de traitement peut traiter les valeurs numériques mesurées, les comparer à des plages de valeurs attendues et afficher en sortie de manière différente, les points où la mesure est dans les plages et les points où la mesure n'est pas dans les plages, par exemple en utilisant des couleurs d'affichage différentes.

Les signaux visuels peuvent compléter l'affichage du modèle décrit ci-avant.

L'invention ne se limite pas aux seuls exemples de réalisation décrits ci-avant en regard des figures, à titre d'exemples illustratifs et non limitatifs.

Notamment, le dispositif médical implantable peut être choisi parmi le groupe comprenant :
- une valve cardiaque,
- un stimulateur cardiaque,
- un implant cochléaire,
- un implant pour la gorge,
- un implant orthopédique,
- un implant cérébral,
- un implant rétinien,
- un cathéter, ou
- un tissu cellulaire (en anglais : « tissue-engineered construct »).

En variante, le dispositif médical peut ne pas être implantable. Il peut alors, notamment, être applicable sur une partie du corps humain. Le dispositif médical peut dans ce cas prendre la forme d'un pansement, d'un bandage ou d'une bande à appliquer sur la peau d'un patient. Le dispositif médical peut également prendre la forme d'une lentille de contact à placer sur la cornée d'un patient.

Enfin, selon une autre variante, le dispositif médical peut n'être ni implantable dans le corps humain, ni applicable sur celui-ci.

## Revendications

1. Dispositif médical (12) comprenant un circuit électrique de mesure (16), dans lequel sont branchés au moins deux capteurs (22) à impédance variable en fonction d'une grandeur physique captée, une source d'énergie électrique (18) pour alimenter le circuit électrique de mesure (16), une antenne (18) pour émettre un champ électromagnétique fonction de l'impédance du circuit électrique de mesure (16), chacun des capteurs (22) étant associé à un interrupteur (24) pour couper l'alimentation en courant du capteur (22) dans ledit circuit de mesure (16), **caractérisé en ce que** le dispositif médical (12) comprend en outre un système (26) de commande des interrupteurs (24) pour successivement commander l'ouverture ou la fermeture des interrupteurs (24), selon des configurations déterminées, dans lequel le système de commande (26) comporte des modules (28 ; 62) disposés en série les uns des autres, chacun commandant un interrupteur (24) associé, les modules (28 ; 62) étant configurés de sorte qu'un changement d'état d'une entrée d'un premier module (28) déclenche la fermeture ou l'ouverture de l'interrupteur (24) associé et déclenche, après un certain temps, un changement d'état à la sortie dudit module (28), correspondant à l'entrée du module (28) suivant de la série de modules.

2. Dispositif selon la revendication **1,** dans lequel chaque module (28 ; 62) comporte un circuit RC série (40, 42) dont la charge ou la décharge provoque la fermeture ou l'ouverture de l'interrupteur (24) associé.

3. Dispositif selon la revendication **2,** dans lequel la charge ou la décharge du circuit RC série d'un module engendre la charge ou la décharge du circuit RC série du module suivant.

4. Dispositif selon la revendication **2** ou **3,** dans lequel chaque module (28) comporte :
∘ un premier transistor (34) dont la source est reliée à l'entrée (46) du module (28), la grille à sa sortie et le drain à la grille de l'interrupteur (24) associé,
∘ un premier inverseur (30, 32) dont l'entrée est reliée à celle du module et la sortie à une première borne (40a) de la résistance (40) du circuit RC série, la deuxième borne (40b) de la résistance (40) étant reliée à une première borne de la capacité (42), la deuxième borne de cette dernière étant reliée à la masse (48),
un deuxième inverseur (36, 38) dont l'entrée est reliée à la deuxième borne (40b) de la résistance (40) et la sortie à celle du module, laquelle sortie constitue l'entrée du module suivant.

5. Dispositif selon la revendication **2** ou **3,** dans lequel chaque module (62) comporte :
o un premier transistor (66) dont la grille est reliée à l'entrée (46) du module et le drain à la borne d'alimentation positive (44),
o un deuxième transistor (24) dont la source est reliée à celle du premier transistor (66), la grille à la sortie du module et le drain à une première borne du capteur (22) à impédance variable dont la deuxième borne est reliée à la masse (48),
∘ une diode (64) dont l'anode est reliée à la source du premier transistor (66) et la cathode à une première borne (40a) de la résistance (40) du circuit RC série, la deuxième borne (40b) de cette dernière étant reliée à la sortie du module, la capacité (42) du circuit RC série étant connectée entre la sortie du module et la masse (48).

6. Dispositif médical selon l'une quelconque des revendications précédentes, dans lequel des ensembles d'un interrupteur (24) et d'un capteur (22) sont montés en série, lesdits ensembles d'un interrupteur (24) et d'un capteur (22) étant montés en parallèle les uns par rapport aux autres pour former une ligne de mesure, le dispositif médical comportant de préférence plusieurs lignes de mesure en parallèle.

7. Dispositif médical selon l'une quelconque des revendications précédentes, dans lequel des ensembles d'un interrupteur (24) et d'un capteur (22) sont montés en parallèle, lesdits ensembles d'un interrupteur (24) et d'un capteur (22) étant montés en série les uns des autres pour former une ligne de mesure, le dispositif médical comportant de préférence plusieurs lignes de mesure en parallèle.

8. Dispositif médical selon l'une quelconque des revendications précédentes, dans lequel au moins un des capteurs (22) est disposé sur une surface extérieure du dispositif médical (12), destinée à être en contact avec une partie du corps sur laquelle le dispositif médical est applicable ou dans laquelle le dispositif médical est implantable, le cas échéant.

9. Dispositif médical selon l'une quelconque des revendications précédentes, dans lequel le circuit de mesure comporte une pluralité d'impédances fixes (60), associées chacune à un interrupteur (24) dont l'ouverture et la fermeture sont commandées par le système de commande (26).

10. Dispositif médical selon l'une quelconque des revendications précédentes, dans lequel le dispositif médical forme un conduit traversant, le dispositif médical comportant, deux capteurs de pression disposés à chacune des extrémités du conduit traversant, dans le conduit traversant.

11. Dispositif médical selon l'une quelconque des revendications précédentes, dans lequel le dispositif médical est un tuteur vasculaire maillé, au moins un capteur (22), de préférence chaque capteur (22), étant disposé au milieu d'un côté d'une maille du tuteur vasculaire.

12. Dispositif médical selon l'une quelconque des revendications précédentes, comprenant une pluralité de lignes de mesure, les lignes de mesure s'étendant sur le dispositif médical, notamment sur le tuteur vasculaire, selon des hélices coaxiales.

13. Système médical comprenant un dispositif médical (12) selon l'une quelconque des revendications précédentes et une unité de réception d'informations depuis le dispositif médical, comprenant des moyens pour capter le champ électromagnétique émis par l'antenne du dispositif médical (12).

14. Système selon la revendication **13,** comprenant en outre une unité d'interrogation (14) du dispositif médical, de préférence confondue avec l'unité de réception d'information, comprenant de préférence des moyens pour émettre un champ électromagnétique adapté à créer un courant induit dans le circuit de mesure du dispositif médical.

15. Système médical selon la revendication précédente et comprenant un dispositif médical (12) selon la revendication 9, comprenant un comparateur (94) destiné à comparer un identifiant (ID1) émis par l'unité d'interrogation (14), avec un code binaire (ID2) associé à une combinaison donnée d'impédances fixes (60).

16. Système selon l'une quelconque des revendications **13** à **15,** comprenant en outre une unité de traitement des informations reçues par l'unité de réception, par exemple un ordinateur, l'unité de traitement des informations présentant de préférence un écran pour afficher en temps réel un modèle du dispositif médical sur lequel sont portées des informations relatives aux valeurs des mesures réalisées à l'aide des capteurs.

17. Procédé d'interrogation d'un dispositif médical selon l'une quelconque des revendications **1** à **12,** ou un système médical selon l'une des revendications **13** à **16,** comprenant les étapes consistant à :
- alimenter le circuit de mesure du dispositif médical en énergie électrique,
- activer le système de commande pour qu'il commande successivement l'ouverture ou la fermeture de chacun des interrupteurs, selon des configurations déterminées, et
- mesurer le champ électromagnétique émis par l'antenne du dispositif médical.

## Patentansprüche

1. Medizinische Vorrichtung (12), die eine elektrische Messschaltung (16) umfasst, in der mindestens zwei Sensoren (22) mit in Abhängigkeit von einer erfassten physikalischen Größe variabler Impedanz, eine elektrische Energiequelle (18), um die elektrische Messschaltung (16) zu versorgen, eine Antenne (18), um ein von der Impedanz der elektrischen Messschaltung (16) abhängiges elektromagnetisches Feld auszustrahlen, angeschlossen sind, wobei jeder der Sensoren (22) einem Schalter (24) zugeordnet ist, um die Stromversorgung des Sensors (22) in der Messschaltung (16) zu unterbrechen,
**dadurch gekennzeichnet, dass** die medizinische Vorrichtung (12) weiter ein System (26) zum Steuern der Schalter (24) umfasst, um nacheinander das Öffnen oder das Schließen der Schalter (24) nach bestimmten Konfigurationen zu steuern, wobei das Steuersystem (26) Module (28; 62) umfasst, die miteinander in Reihe angeordnet sind, wobei jedes einen zugehörigen Schalter (24) steuert, wobei die Module (28; 62) so ausgelegt sind, dass ein Zustandswechsel eines Eingangs eines ersten Moduls (28) das Schließen oder das Öffnen des zugehörigen Schalters (24) auslöst, und nach einer gewissen Zeit einen Zustandswechsel am Ausgang des Moduls (28), der dem Eingang des nächsten Moduls (28) der Modulreihe entspricht, auslöst.

2. Vorrichtung nach Anspruch **1,** wobei jedes Modul (28; 62) eine RC-Reihenschaltung (40, 42) umfasst, deren Laden oder deren Entladen das Schließen oder das Öffnen des zugehörigen Schalters (24) bewirkt.

3. Vorrichtung nach Anspruch **2,** wobei das Laden oder das Entladen der RC-Reihenschaltung eines Moduls das Laden oder das Entladen der RC-Reihenschaltung des nächsten Moduls hervorruft.

4. Vorrichtung nach Anspruch **2** oder **3,** wobei jedes Modul (28) umfasst:
- einen ersten Transistor (34), dessen Quelle an den Eingang (46) des Moduls (28), dessen Gatter an seinem Ausgang, und dessen Senke an das Gatter des zugehörigen Schalters (24) angeschlossen ist,
- einen ersten Inverter (30, 32), dessen Eingang an den des Moduls und dessen Ausgang an einen ersten Anschluss (40a) des Widerstands (40) der RC-Reihenschaltung angeschlossen ist, wobei der zweite Anschluss (40b) des Widerstands (40) an einen ersten Anschluss der Kapazität (42) angeschlossen ist, wobei der zweite Anschluss der letzteren an die Masse (48) angeschlossen ist,
einen zweiten Inverter (36, 38), dessen Eingang an den zweiten Anschluss (40b) des Widerstands (40) und dessen Ausgang an den des Moduls angeschlossen ist, wobei der Ausgang den Eingang des nächsten Moduls darstellt.

5. Vorrichtung nach Anspruch **2** oder **3,** wobei jedes Modul (62) umfasst:
- einen ersten Transistor (66), dessen Gatter an den Eingang (46) des Moduls und dessen Senke an den positiven Versorgungsanschluss (44) angeschlossen ist,
- einen zweiten Transistor (24), dessen Quelle an die des ersten Transistors (66), dessen Gatter an den Ausgang des Moduls, und dessen Senke an einen ersten Anschluss des Sensors (22) mit variabler Impedanz angeschlossen ist, dessen zweiter Anschluss an die Masse (48) angeschlossen ist,
- eine Diode (64), deren Anode an die Quelle des ersten Transistors (66) und deren Kathode an einen ersten Anschluss (40a) des Widerstands (40) der RC-Reihenschaltung angeschlossen ist, wobei der zweite Anschluss (40b) des letzteren an den Ausgang des Moduls angeschlossen ist, wobei die Kapazität (42) der RC-Reihenschaltung mit dem Ausgang des Moduls und der Masse (48) verbunden ist.

6. Medizinische Vorrichtung nach einem der vorstehenden Ansprüche, wobei Baugruppen aus einem Schalter (24) und einem Sensor (22) in Reihe geschaltet sind, wobei die Baugruppen aus einem Schalter (24) und einem Sensor (22) in Bezug zueinander parallelgeschaltet sind, um eine Messleitung zu bilden, wobei die medizinische Vorrichtung vorzugsweise mehrere parallele Messleitungen umfasst.

7. Medizinische Vorrichtung nach einem der vorstehenden Ansprüche, wobei Baugruppen aus einem Schalter (24) und einem Sensor (22) parallelgeschaltet sind, wobei die Baugruppen aus einem Schalter (24) und einem Sensor (22) miteinander in Reihe geschaltet sind, um eine Messleitung zu bilden, wobei die medizinische Vorrichtung vorzugsweise mehrere parallele Messleitungen umfasst.

8. Medizinische Vorrichtung nach einem der vorstehenden Ansprüche, wobei mindestens einer der Sensoren (22) an einer Außenfläche der medizinischen Vorrichtung (12), die dazu bestimmt ist, sich mit einem Teil des Körpers, an dem die medizinische Vorrichtung angewendet werden kann oder in den die medizinische Vorrichtung gegebenenfalls implantiert werden kann, in Kontakt zu befinden, angeordnet ist.

9. Medizinische Vorrichtung nach einem der vorstehenden Ansprüche, wobei die Messschaltung eine Vielzahl von festen Impedanzen (60) umfasst, die jeweils einem Schalter (24) zugeordnet sind, dessen Öffnen und dessen Schließen vom Steuersystem (26) gesteuert werden.

10. Medizinische Vorrichtung nach einem der vorstehenden Ansprüche, wobei die medizinische Vorrichtung einen Durchgangskanal bildet, wobei die medizinische Vorrichtung zwei Drucksensoren umfasst, die an jedem der Enden des Durchgangskanals im Durchgangskanal angeordnet sind.

11. Medizinische Vorrichtung nach einem der vorstehenden Ansprüche, wobei es sich bei der medizinischen Vorrichtung um eine Gefäßstütze mit Maschen handelt, wobei mindestens ein Sensor (22), vorzugsweise jeder Sensor (22) in der Mitte einer Seite einer Masche der Gefäßstütze angeordnet ist.

12. Medizinische Vorrichtung nach einem der vorstehenden Ansprüche, die eine Vielzahl von Messleitungen umfasst, wobei sich die Messleitungen an der medizinischen Vorrichtung, insbesondere an der Gefäßstütze, gemäß koaxialen Schraubenlinien erstrecken.

13. Medizinisches System, das eine medizinische Vorrichtung (12) nach einem der vorstehenden Ansprüche und eine Einheit zum Empfangen von Informationen von der medizinischen Vorrichtung umfasst, die Mittel umfasst, um das von der Antenne der medizinischen Vorrichtung (12) ausgestrahlte elektromagnetische Feld zu erfassen.

14. System nach Anspruch **13,** das weiter eine Einheit zum Abfragen (14) der medizinischen Vorrichtung, vorzugsweise mit der Informationsempfangseinheit zusammengefasst, umfasst, die vorzugsweise Mittel zum Ausstrahlen eines elektromagnetischen Feldes, das geeignet ist, in der Messschaltung der medizinischen Vorrichtung einen induzierten Strom zu erzeugen, umfasst.

15. Medizinisches System nach dem vorstehenden Anspruch, und eine medizinische Vorrichtung (12) nach Anspruch 9 umfassend, die einen Komparator (94) umfasst, der dazu bestimmt ist, eine von der Abfrageeinheit (14) ausgestrahlte Kennung (ID1) mit einem binären Code (ID2), der einer gegebenen Kombination von festen Impedanzen (60) zugeordnet ist, zu vergleichen.

16. System nach einem der Ansprüche **13** bis **15,** das weiter eine Einheit zum Verarbeiten der Informationen, die von der Empfangseinheit empfangen werden, zum Beispiel einen Computer, umfasst, wobei die Einheit zum Verarbeiten der Informationen vorzugsweise einen Bildschirm aufweist, um in Echtzeit ein Modell der medizinischen Vorrichtung anzuzeigen, in das Informationen, die sich auf die Werte der mithilfe der Sensoren ausgeführten Messungen beziehen, übertragen sind.

17. Verfahren zum Abfragen einer medizinischen Vorrichtung nach einem der Ansprüche **1** bis **12** oder eines medizinischen Systems nach einem der Ansprüche **13** bis **16,** das die Schritte umfasst, bestehend im:
- Versorgen der Messschaltung der medizinischen Vorrichtung mit elektrischer Energie,
- Aktivieren des Steuersystems, damit es nacheinander das Öffnen oder das Schließen jedes der Schalter nach bestimmten Konfigurationen steuert, und
- Messen des von der Antenne der medizinischen Vorrichtung ausgestrahlten elektromagnetischen Feldes.

## Claims

1. Medical device (12) comprising an electrical measurement circuit (16), wherein are connected at least two sensors (22) the impedance of which varies as a function of a sensed physical quantity, an electrical power source (18) for supplying power to the electrical measurement circuit (16), an antenna (18) for emitting an electromagnetic field according to the impedance of the electrical measurement circuit (16), each of the sensors (22) being associated with a switch (24) for interrupting the current supply of the sensor (22) in said measurement circuit (16), **characterized in that** the medical device (12) further comprises a control system (26) of the switches (24) to successively control the opening or the closing of the switches (24), according to determined configurations, wherein the control system (26) includes modules (28; 62) arranged in series with respect to one another, each controlling an associated switch (24), the modules (28; 62) being configured so that a change of state of an input of a first module (28) triggers the closing or opening of the associated switch (24) and triggers, after an amount of time, a change of state at the output of said module (28), corresponding to the input of the next module (28) of the series of modules.

2. Medical device according to claim **1,** wherein each module (28; 62) includes a series RC circuit (40, 42) the charging or discharging of which triggers the closing or opening of the associated switch (24).

3. Medical device according to claim **2,** wherein charging or discharging of a series RC circuit of a module induces the charging or discharging of the series RC circuit of the next module.

4. Medical device according to one of claims **2** or **3,** wherein each module (28) includes:
∘ a first transistor (34) the source of which is connected to the input (46) of the module (28), the gate to the output thereof and the drain to the gate of the associated switch (24),
∘ a first inverter (30, 32) the input of which is connected to that of the module and the output to a first terminal (40a) of the resistor (40) of the series RC circuit, the second terminal (40b) of the resistor (40) being connected to a first terminal of the capacitor (42), the second terminal thereof being connected to the ground (48),
∘ a second inverter (36, 38) the input of which is connected to the second terminal (40b) of the resistor (40) and the output to that of the module, said output forming the input of the next module.

5. Medical device according to one of claims **2** or **3,** wherein each module (62) includes:
∘ a first transistor (66) the gate of which is connected to the input (46) of the module and the drain to the positive power supply terminal (44),
∘ a second transistor (24) the source of which is connected to that of the first transistor (66), the gate to the output of the module and the drain to a first terminal of the variable-impedance sensor (22) of which the second terminal is connected to the ground (48),
∘ a diode (64) of which the anode is connected to the source of the first transistor (66) and the cathode to a first terminal (40a) of the resistor (40) of the series RC circuit, the second terminal (40b) of the latter being connected to the output of the module, the capacitor (42) of the series RC circuit being connected between the output of the module and the ground (48).

6. Medical device according to any one of the preceding claims, wherein sets of a switch (24) and a sensor (22) are mounted in series, said sets of a switch (24) and a sensor (22) being mounted in parallel with respect to one another to form a measurement line, the medical device preferably including a plurality of measurement lines in parallel.

7. Medical device according to any one of the preceding claims, wherein sets of a switch (24) and a sensor (22) are mounted in parallel, said sets of a switch (24) and a sensor (22) being mounted in series with respect to one another to form a measurement line, the medical device preferably including a plurality of measurement lines in parallel.

8. Medical device according to any one of the preceding claims, wherein at least one of the sensors (22) is arranged on an outer surface of the medical device (12), intended to be in contact with a part of the body on which the device is applied or in which the device is implantable, if applicable.

9. Medical device according to any one of the preceding claims, wherein the measurement circuit includes a plurality of fixed impedances (60), each associated with a switch (24) of which the opening and closing are controlled by the control system (26).

10. Medical device according to any one of the preceding claims, wherein the medical device forms a through conduit, the medical device including two pressure sensors arranged at each of the ends of the through conduit, in the through conduit.

11. Medical device according to any one of the preceding claims, wherein the medical device is a meshed vascular stent, at least one sensor (22), preferably each sensor (22), being arranged at the mid-point of one side of a mesh of the vascular stent.

12. Medical device according to any one of the preceding claims, comprising a plurality of measurement lines, the measurement lines extending on the medical device, particularly on the vascular stent, along coaxial helices.

13. Medical system comprising a medical device (12) according to any one of the preceding claims and a unit for receiving information from the medical device, comprising means for sensing the electromagnetic field emitted by the antenna of the medical device (12).

14. Medical system according to claim **13,** further comprising a querying unit (14) of the medical device, preferably merged with the unit for receiving information, preferably comprising means for emitting an electromagnetic field suitable for creating an induced current in the measurement circuit of the medical device.

15. Medical system according to the preceding claim and comprising a medical device (12) according to claim 9, comprising a comparator (94) intended to compare an identifier (ID1) emitted by the querying unit (14), with a binary code (ID2) associated with a given combination of fixed impedances (60).

16. Medical system according to any one of claims **13** to **15,** further comprising a unit for processing the information received by the reception unit, for example a computer, the unit for processing information preferably having a screen to display in real time a model of the medical device on which is transferred information relating to the values of the measurements made using the sensors.

17. Method for querying a medical device according to any one of claims **1** to **12,** or a medical system according to one of claims **13** to **16,** comprising steps consisting of:
- powering the measurement circuit of the medical device,
- activating the control system so that it successively controls the opening or closing of each of the switches, according to determined configurations, and
- measuring the electromagnetic field emitted by the antenna of the medical device.
